# EUROPÄISCHE PATENTSCHRIFT

(11) **EP 3 212 242 B1**
(45) Veröffentlichungstag und Bekanntmachung des Hinweises auf die Patenterteilung: **23.08.2023**
(21) Anmeldenummer: 15787963.6
(22) Anmeldetag: 27.10.2015
(51) Int. Cl.: A61L 2/06, A61L 11/00, B09B 3/00, F23G 5/46, F23G 5/02, B09B 3/40

(54) **VERFAHREN UND VORRICHTUNG ZUR HYGIENISIERUNG VON BIOABFÄLLEN MIT ABGASEN AUS VERBRENNUNGSEINRICHTUNGEN**
METHOD AND DEVICE FOR SANITIZING BIOGENIC WASTES WITH WASTE GASES FROM INCINERATION DEVICES
PROCÉDÉ ET DISPOSITIF DE DÉSINFECTION DE DÉCHETS ORGANIQUES PAR DES GAZ DE COMBUSTION ISSUS DE DISPOSITIFS D'INCINÉRATION

(30) Priorität: 27.10.2014 DE 102014221846
(43) Veröffentlichungstag der Anmeldung: 06.09.2017
(73) Patentinhaber: Rosmarin Holdings Limited, Ramsey IM8 2LQ (IM)
(72) Erfinder: ANIOL, Stefan, 80999 München (DE); OELKERS, Martin, 85244 Röhrmoos (DE); UHLIG, Julien, 85276 Pfaffenhofen a.d. Ilm (DE)
(74) Vertreter: Ernicke, Moritz
(86) Internationale Anmeldenummer: PCT/EP2015/074834
(87) Internationale Veröffentlichungsnummer: WO 2016/066626

(56) Entgegenhaltungen:
- WO-A2-2006/029460
- WO-A2-2008/057467
- DE-A1- 4 117 420
- DE-A1- 10 046 389
- JP-A- 2000 272 708
- US-A- 5 020 321

## Beschreibung

Die Erfindung betrifft ein Verfahren zur Hygienisierung von Bioabfällen mit Abgasen aus Verbrennungseinrichtungen nach Anspruch 1 sowie eine Vorrichtung zur Durchführung dieses Verfahrens nach Anspruch 8.

Aus der Schrift WO 2006/029460 A2 ist ein Verfahren zur Sterilisierung von Abfällen in einer mobilen Mülleinsammelvorrichtung bekannt.

In der Schrift JP 2000 272708 A wird ein Müllfahrzeug offenbart, dass dazu ausgebildet ist, eingesammelten Müll durch Erhitzen zu verkleinern. Das Müllfahrzeug dient zum Transport und zur vorbereitenden Behandlung von Abfällen, die auch Lebensmittelabfälle umfassen können und die zu einer Müllverbrennungslage befördert werden. Das Fahrzeug soll bereits auf dem Transportweg einen Erwärmungs- und Volumenreduzierungsprozess durchführen. Das Fahrzeug nutzt Abgas als Wärmequelle für diesen Prozess. Der Abfall wird in einer abschließbaren Verarbeitungskammer gesammelt, die als ein Autoklav ausgebildet ist. In dem Autoklav werden die Abfälle mit eingeblasener heißer Luft eingeschlossen. In der Verarbeitungskammer ist weiterhin ein Wärmetauscher für die Zuführung weiterer Wärmeenergie aus einem Flüssigkeitskreislauf vorgesehen, der den Wärmetauscher durchströmt und an anderer Stelle von dem Abgas erwärmt wird. Das Abgas kommt nicht in Kontakt mit den Abfällen. Der eingeschlossene Abfall wird unter hohem Druck behandelt, was die erwünschte Reduzierung des Volumens erzeugt. Die Abfälle werden anschließend an einer Müllverbrennungsanlage abgeladen.

In Schwellenländern und Ländern der dritten Welt stellen Bioabfälle aufgrund ihrer Kontamination mit Bakterien und Keimen häufig eine "Gefahr für das Grundwasser" dar. Es ist daher Aufgabe der vorliegenden Erfindung ein einfaches und ohne großen technischen Aufwand durchführbares Verfahren zur Hygienisierung von Bioabfällen sowie eine Vorrichtung zur Durchführung dieses Verfahrens anzugeben.

Die Lösung dieser Aufgabe erfolgt durch die Merkmale des Anspruchs 1 und 8.

Eine oder mehrere verschließbare Hygienisierungskammern werden mit Bioabfällen bestückt. Die Hygienisierungskammer wird verschlossen und heiße Abgase (zwischen 80°C und 140°C) werden solange durch die Hygienisierungskammer(n) geführt, bis Keime und Bakterien in den Bioabfällen abgetötet sind.

Gemäß einer vorteilhaften Weiterbildung kann zusätzlich oder alternativ auch Abwärme aus einer Verbrennungseinrichtung über einen Flüssigkeitskreislauf in die wenigstens eine Hygienisierungskammer eingekoppelt werden. Auch durch diese Erwärmung der Bioabfälle werden Keime und Bakterien abgetötet.

Durch die Hygienisierung der Bioabfälle werden diese als Quelle für Krankheiten und Grundwasserverseuchung ausgeschaltet. Die Erfindung ist für dezentrale Standorte z.B. in Drittwelt- oder Schwellenländern oder Orten mit Inselnetzen geeignet. Sie kann sowohl stationär als auch mobil ausgelegt sein.

Bei Übertemperatur in der Kammer wird das Abgas direkt in den Kamin geleitet. Die Regulierung erfolgt automatisch oder manuell.

Explosionsschutz in den Kammern durch Inertisierung mit CO2-haltigem Abgas. Das bei der Vergärung des Bioabfalls austretende explosive Gas (CH4) bekommt keinen Sauerstoff und wird durch strömendes Abgas aus dem Raum gespült.

Bioabfall wird inkl. Tonnel Behälter in die Kammer gestellt - es gibt kein Handling mit losen Bioabfällen. Kein Radlader oder andere Maschinen sind nötig.

Zusätzliches Wasser zum besseren Wärmeübergang wird nicht beigegeben. Die Verweilzeit wird den Bedürfnissen angepasst.

Die zu befüllende Kammer ist offen und frei von Abgasen. Sie wird vor der Öffnung mit Luft gespült. Eine Exposition der Arbeiter in giftiger und erstickender Atmosphäre ist durch CO/ CO2-Warner ausgeschlossen.

Der Bioabfall bekommt durch den Wärmeeintrag ein höheren TS-Gehalt, wird etwas angetrocknet, was die Transportfähigkeit verbessert. Das ist besonders in Gegenden mit schwierigen Straßenverhältnissen von Vorteil.

Der Bioabfall kann nach der Hygienisierung kompostiert, fermentiert oder direkt als Bodenverbesserer genutzt werden.

Bewegliche Teile sind nicht vorhanden. Lediglich die Abfallbehälter werden bewegt. Durch die Nutzung des Abgases wird der Gesamtwirkungsgrad der Energieanlage erhöht.

### Varianten:

Abgas aus:
- BHKW einer Biomasse-Vergasung
- Erdgas-BHKWs, Dieselmotoren und - generatoren
- Heizungen oder anderen Verbrennungseinrichtungen

Genutzt wird die Restwärme > 70 °C und das sauerstoffarme Gas zur Inertisierung und Spülung.

Kammern:
- in kompletten oder intern unterteilten Containern
- in sonstigen Räumen oder Schränken
gefordert wird pro Kammer:
- eine Zuleitung mit Abgas
- optional eine weitere Wärmequelle z.B. Kühlwasser aus BHKW
- sichere Ableitung des Abgases über z. B. Kamin
- Abdichtung gegenüber anderen Räumen
- Sicherheitstechnik gegen unbefugtes Betreten der Kammer bzw. um eine Exposition in giftiger und erstickender Atmosphäre auszuschließen: mindestens CO/ CO2 Warner mit Blinklicht und Hupe; ggf. Türschliesserkontakt.

### Weitere Optionen der Abgasnutzung im Zusammenhang mit Bioabfällen: Trocknung

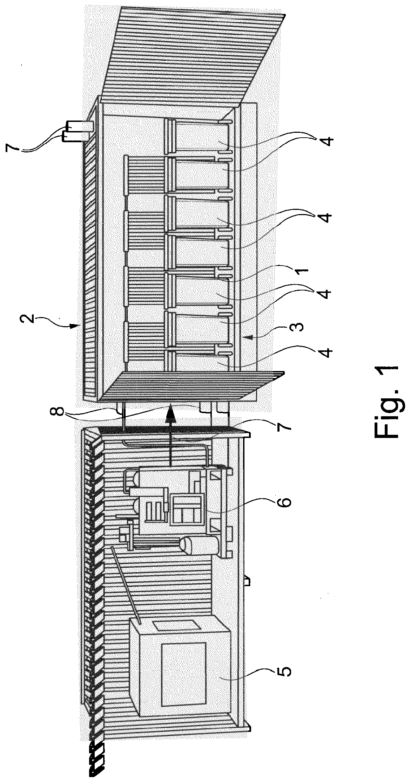

Fig. 1 zeigt eine beispielhafte Ausgestaltung der Erfindung mit zwei Hygienisierungskammern 1, 2 in einem Standardcontainer 3. Das Abgas 7 und die Abwärme 8 zur Hygienisierung der Bioabfälle 4 wird durch eine Vorrichtung zur Holzvergasung 5 und ein damit betriebenes BHKW 6 bereitgestellt.

## Patentansprüche

1. Verfahren zur Hygienisierung von Bioabfällen (4) mit Abgasen (7) aus Verbrennungseinrichtungen (5,6), mit den Verfahrensschritten:
- Einbringen der Bioabfälle (4) in wenigstens eine Hygienisierungskammer (1,2);
- Einkoppeln von Abwärme (8) aus einer Verbrennungseinrichtung (5,6)über einen Flüssigkeitskreislauf in die wenigstens eine Hygienisierungskammer (1,2);
**gekennzeichnet durch**:
- Durchströmen der Hygienisierungskammer (1,2) mit heißem Abgas (7) aus der Verbrennungseinrichtung (5,6) und zwar solange bis durch die Temperatur und die Toxizität der Abgase (7) in den Bioabfällen (4) enthaltene Keime und Bakterien abgetötet sind.

2. Verfahren nach Anspruch 1, **dadurch gekennzeichnet, dass** die wenigstens eine Hygienisierungskammer (1,2) nach der Hygienisierung und vor dem Öffnen mit Frischluft gespült wird.

3. Verfahren nach Anspruch 1 oder 2, **dadurch gekennzeichnet, dass** das heiße Abgas (7) aus einer Verbrennungseinrichtung (5,6) vor der Zuführung zu der wenigstens einen Hygienisierungskammer (1,2) auf eine Temperatur im Bereich zwischen 80°C und 140°C und insbesondere im Bereich zwischen 90°C und 130°C gekühlt wird.

4. Verfahren nach einem der vorhergehenden Ansprüche, **dadurch gekennzeichnet, dass** die zu hygienisierenden Bioabfälle (4) in offenen Behältern in die wenigstens eine Hygienisierungskammer (1,2) eingebracht werden.

5. Verfahren nach einem der vorhergehenden Ansprüche, **dadurch gekennzeichnet, dass** wenigstens zwei Hygienisierungskammern (1,2) vorgesehen sind, die im Batchtrieb gefahren werden.

6. Verfahren nach einem der vorhergehenden Ansprüche, **dadurch gekennzeichnet, dass** die wenigstens eine Hygienisierungskammer (1,2) mobil ausgestaltet ist.

7. Verfahren nach einem der vorhergehenden Ansprüche, **dadurch gekennzeichnet, dass** das Durchströmen mit heißem Abgas (7) und das Einkoppeln von Abwärme (8) solange fortgesetzt wird, bis die Bioabfälle (4) einen vorbestimmten Trocknungszustand erreicht haben.

8. Vorrichtung zur Hygienisierung von Bioabfällen (4) mit Abgasen (7) aus Verbrennungseinrichtungen (5,6), insbesondere zur Durchführung des Verfahrens nach einem der vorhergehenden Ansprüche, wobei die Vorrichtung ausgebildet ist, mit:
- wenigstens einer Hygienisierungskammer (1,2), die eine verschließbare Öffnung zum Einbringen und Ausbringen von zu hygienisierenden Bioabfällen (4), einen Abgaseinlass und einen Abgasauslass aufweist;
- wobei in der wenigstens einen Hygienisierungskammer (1,2) eine Wärmetauschereinrichtung zum Einkoppeln von Wärme (8) in die Hygienisierungskammer (1,2) mittels eines Flüssigkeitskreislaufs vorgesehen ist und
- der Flüssigkeitskreislauf mit dem Kühlkreislauf einer Verbrennungseinrichtung (5,6) verbunden ist.

9. Vorrichtung nach Anspruch 8, **dadurch gekennzeichnet, dass** die wenigstens eine Hygienisierungskammer (1,2) eine Frischluftzuführeinrichtung umfasst.

10. Vorrichtung nach Anspruch 8 oder 9, **dadurch gekennzeichnet, dass** die Verbrennungseinrichtung (5,6) ein BHKW, ein Verbrennungsmotor oder eine Verbrennungsheizung ist.

11. Vorrichtung nach einem der vorhergehenden Ansprüche 8 bis 10, **dadurch gekennzeichnet, dass** zwei Hygienisierungskammern (1,2) nebeneinander in einem Standard-Container (3) angeordnet sind.

12. Vorrichtung nach einem der vorhergehenden Ansprüche 8 bis 11, **dadurch gekennzeichnet, dass** die Verbrennungseinrichtung (5,6) in einem Standard-Container (3) angeordnet ist.

## Claims

1. Method for the hygienization of biological waste (4) with offgases (7) from combustion devices (5, 6), comprising the method steps:
- introducing the biological waste (4) into at least one hygienization chamber (1, 2);
- coupling waste heat (8) from a combustion device (5, 6) into the at least one hygienization chamber (1, 2) via a liquid circuit;
**characterized by**:
- flowing hot offgas (7) from the combustion device (5, 6) through the hygienization chamber (1, 2), specifically until germs and bacteria contained in the biological waste (4) have been killed by the temperature and the toxicity of the offgases (7) .

2. Method according to Claim 1, **characterized in that** the at least one hygienization chamber (1, 2) is flushed with fresh air after the hygienization and prior to opening.

3. Method according to Claim 1 or 2, **characterized in that**, prior to being fed to the at least one hygienization chamber (1, 2), the hot offgas (7) from a combustion device (5, 6) is cooled to a temperature in the range between 80°C and 140°C and in particular in the range between 90°C and 130°C.

4. Method according to one of the preceding claims, **characterized in that** the biological waste (4) to be hygienized is introduced in open containers into the at least one hygienization chamber (1, 2).

5. Method according to one of the preceding claims, **characterized in that** at least two hygienization chambers (1, 2) are provided, which are run in batch mode.

6. Method according to one of the preceding claims, **characterized in that** the at least one hygienization chamber (1, 2) is of mobile design.

7. Method according to one of the preceding claims, **characterized in that** the flowing-through with hot offgas (7) and the coupling-in of waste heat (8) is continued until the biological waste (4) has reached a predetermined drying state.

8. Apparatus for the hygienization of biological waste (4) with offgases (7) from combustion devices (5, 6), in particular for carrying out the method according to one of the preceding claims, wherein the apparatus is configured with:
- at least one hygienization chamber (1, 2) which comprises a closable opening for introducing and discharging biological waste (4) to be hygienized, an offgas inlet and an offgas outlet;
- wherein a heat exchanger device for coupling heat (8) into the hygienization chamber (1, 2) by means of a liquid circuit is provided in the at least one hygienization chamber (1, 2), and
- the liquid circuit is connected to the cooling circuit of a combustion device (5, 6).

9. Apparatus according to Claim 8, **characterized in that** the at least one hygienization chamber (1, 2) comprises a fresh-air feed device.

10. Apparatus according to Claim 8 or 9, **characterized in that** the combustion device (5, 6) is a combined heat and power unit, a combustion engine or a combustion heater.

11. Apparatus according to one of the preceding Claims 8 to 10, **characterized in that** two hygienization chambers (1, 2) are arranged next to one another in a standard container (3).

12. Apparatus according to one of the preceding Claims 8 to 11, **characterized in that** the combustion device (5, 6) is arranged in a standard container (3).

## Revendications

1. Procédé d'hygiénisation de déchets biologiques (4) avec des gaz d'échappement (7) provenant d'appareils de combustion (5, 6), avec les étapes de procédé suivantes :
- l'introduction des déchets biologiques (4) dans au moins une chambre d'hygiénisation (1, 2) ;
- l'injection de chaleur résiduaire (8) provenant d'un appareil de combustion (5, 6) par l'intermédiaire d'un circuit de liquide dans l'au moins une chambre d'hygiénisation (1, 2) ;
**caractérisé par** :
- le brassage de la chambre d'hygiénisation (1, 2) avec du gaz d'échappement chaud (7) provenant de l'appareil de combustion (5, 6), et ce jusqu'à ce que les germes et les bactéries contenus dans les déchets biologiques (4) soient détruits par la température et la toxicité des gaz d'échappement (7).

2. Procédé selon la revendication 1, **caractérisé en ce que** l'au moins une chambre d'hygiénisation (1, 2) est rincée avec de l'air frais après l'hygiénisation et avant l'ouverture.

3. Procédé selon la revendication 1 ou 2, **caractérisé en ce que** le gaz d'échappement chaud (7) provenant d'un appareil de combustion (5, 6) est refroidi à une température dans la plage comprise entre 80 °C et 140 °C, et notamment dans la plage comprise entre 90 °C et 130 °C, avant d'être acheminé vers l'au moins une chambre d'hygiénisation (1, 2).

4. Procédé selon l'une quelconque des revendications précédentes, **caractérisé en ce que** les déchets biologiques (4) à hygiéniser sont introduits dans des récipients ouverts dans l'au moins une chambre d'hygiénisation (1, 2).

5. Procédé selon l'une quelconque des revendications précédentes, **caractérisé en ce qu'**au moins deux chambres d'hygiénisation (1, 2) sont prévues, qui sont pilotées en mode discontinu.

6. Procédé selon l'une quelconque des revendications précédentes, **caractérisé en ce que** l'au moins une chambre d'hygiénisation (1, 2) est conçue sous forme mobile.

7. Procédé selon l'une quelconque des revendications précédentes, **caractérisé en ce que** le brassage avec du gaz d'échappement chaud (7) et l'injection de chaleur résiduaire (8) se poursuivent jusqu'à ce que les déchets biologiques (4) atteignent un état de séchage prédéterminé.

8. Dispositif pour l'hygiénisation de déchets biologiques (4) avec des gaz d'échappement (7) provenant d'appareils de combustion (5, 6), notamment pour la réalisation du procédé selon l'une quelconque des revendications précédentes, le dispositif étant configuré avec :
- au moins une chambre d'hygiénisation (1, 2), qui présente une ouverture pouvant être fermée pour l'introduction et l'évacuation de déchets biologiques (4) à hygiéniser, une entrée de gaz d'échappement et une sortie de gaz d'échappement ;
- dans l'au moins une chambre d'hygiénisation (1, 2), un appareil d'échange de chaleur pour injecter de la chaleur (8) dans la chambre d'hygiénisation (1, 2) au moyen d'un circuit de liquide étant prévu, et
- le circuit de liquide étant relié au circuit de refroidissement d'un appareil de combustion (5, 6).

9. Dispositif selon la revendication 8, **caractérisé en ce que** l'au moins une chambre d'hygiénisation (1, 2) comprend un appareil d'acheminement d'air frais.

10. Dispositif selon la revendication 8 ou 9, **caractérisé en ce que** l'appareil de combustion (5, 6) est une centrale de cogénération, un moteur à combustion ou un chauffage à combustion.

11. Dispositif selon l'une quelconque des revendications 8 à 10 précédentes, **caractérisé en ce que** deux chambres d'hygiénisation (1, 2) sont agencées côte à côte dans un conteneur standard (3).

12. Dispositif selon l'une quelconque des revendications précédentes 8 à 11, **caractérisé en ce que** l'appareil de combustion (5, 6) est agencé dans un conteneur standard (3).
